Europäisches Patentamt

European Patent Office

Office européen des brevets

(11) Numéro de publication: **0 356 325 B1**

# FASCICULE DE BREVET EUROPEEN

(45) Date de publication de fascicule du brevet: **04.05.94**

(51) Int. Cl.5: **A61K 47/00**

(21) Numéro de dépôt: **89402306.8**

(22) Date de dépôt: **18.08.89**

Le dossier contient des informations techniques présentées postérieurement au dépôt de la demande et ne figurant pas dans le présent fascicule.

(54) **Composition pharmaceutique comportant un principe actif peu soluble dans l'eau et au moins un glycéride gélifié par au moins un polymère cellulosique.**

(30) Priorité: **19.08.88 FR 8811037**

(43) Date de publication de la demande:
**28.02.90 Bulletin 90/09**

(45) Mention de la délivrance du brevet:
**04.05.94 Bulletin 94/18**

(84) Etats contractants désignés:
**AT BE CH DE GB IT LI LU NL SE**

(56) Documents cités:

**CHEMICAL ABSTRACTS, vol. 98, no. 4, 24 janvier, page 95, résumé no. 18432a, Columbus, Ohio, US; T. ECKERT et al.: "Transparent oleogels on the basis of polyacrylic acid - Carbopol 934", & FETTE, SEIFEN, ANSTRICHM. 1982, 84(11),451-2**

**JOURNAL OF PHARMACEUTICAL SCIENCES, vol. 72, no. 4, avril 1983, pages 454-458, American Pharmaceutical Association; I. UEDA et al.: "Effect of ethyl cellulose in a medium-chain triglyceride on the bioavailability of ceftizoxime"**

(73) Titulaire: **Aiache, Jean-Marc**
**17, rue du Maréchal Galliéni**
**F-63000 Clermont-Ferrand(FR)**

(72) Inventeur: **Aiache, Jean-Marc**
**17, rue du Maréchal Galliéni**
**F-63000 Clermont-Ferrand(FR)**

(74) Mandataire: **Ahner, Francis et al**
**CABINET REGIMBEAU**
**26, avenue Kléber**
**F-75116 Paris (FR)**

Rank Xerox (UK) Business Services
(3.10/3.09/3.3.3)

## Description

La présente invention concerne une composition pharmaceutique stable et concentrée utilisable en médecine humaine ou vétérinaire et comportant un principe actif peu soluble dans l'eau, en combinaison avec un véhicule particulier lui conférant une consistance adaptable à toutes les voies d'administration thérapeutique et évitant toute forme d'irritation.

On a ressenti le besoin d'une composition stable et concentrée pour de nombreux principes actifs, et notamment pour un antibiotique bien connu : la doxycycline.

En effet, aucune des compositions antibiotiques à base de doxycycline actuellement connue ou commercialisée ne réunit toutes les qualités nécessaires à une administration orale, topique ou parentérale chez l'homme ou chez l'animal. Une formulation concentrée idéale de doxycycline est une formulation :
- administrable de façon pratique par voie orale, topique ou parentérale,
- bien tolérée,
- compatible avec l'effet prolongé de l'antibiotique,
- stable dans le temps.

Une telle formulation n'existe pas dans la technique antérieure.

C'est ainsi que l'administration parentérale nécessite des formulations concentrées d'un tel principe actif, à savoir des formulations comportant au moins 15 % en poids de doxycycline. De telles formulations concentrées n'ont pas encore pu être réalisées du fait de l'irritation locale, de la faible solubilité et de l'instabilité de la molécule.

Le brevet US 3 674 859 revendique une solution aqueuse comportant 1 à 15 % en poids de doxycycline, mais il ressort de la description et des exemples que les formulations selon ce brevet comportent en fait seulement 2 à 8 % en poids de doxycycline.

En outre, aucun document de la technique antérieure ne divulgue la possibilité d'une administration intra-musculaire ou sous-cutanée.

Quant aux formes d'administration topique et per os qui existent dans la technique antérieure, elles se sont avérées entraîner des irritations locales importantes, souvent liées au sel de doxycycline ; on a notamment rapporté des incidents d'oesophagite à la suite d'ingestion, par l'homme, de comprimés ou de gélules comportant de la doxycycline.

Des problèmes du même type se posent avec d'autres principes actifs instables en milieu aqueux tels que le paracétamol, le pamoate de pyrantel, l'amoxycilline.

C'est pourquoi, la présente invention concerne une composition pharmaceutique stable, administrable par voie orale, topique ou parentérale chez l'homme ou l'animal, comportant un principe actif peu soluble dans l'eau à raison d'une quantité pouvant atteindre 20 % en poids voire 25 % en poids et au moins un glycéride gélifié par au moins un polymère cellulosique. Le polymère cellulosique est de préférence présent à raison d'une quantité comprise entre 1 et 10 % en poids.

De préférence, le principe actif choisi est la doxycycline. Il s'agit d'un antibiotique répondant à la formule $(C_{22}H_{24}N_2O_8, H_2O)$. Différents sels de doxycycline peuvent être utilisés dans le cadre de la présente invention. On utilise de préférence l'hyclate de doxycycline, à savoir le sel répondant à la formule $[(C_{22}H_{24}N_2O_8, HCl)_2, C_2H_6O, H_2O]$.

Mais d'autres principes actifs peuvent être utilisés dans la composition pharmaceutique de la présente invention. On peut notamment citer, à ce titre, l'acide oxolinique, l'amoxicilline, le pyrantel notamment sous forme pamoate, la furazolidone, le paracetamol et les mélanges vitaminiques.

L'article de VEDA paru dans Journal of Pharmaceutical Sciences, Vol. 72 N°. 4, Avril 1983, pp. 454-458 a étudié l'effet de l'éthyl cellulose combiné à un triglycéride, le Miglyol® 812 sur la biodisponibilité de la ceftizoxime. Toutefois, la composition obtenue ne comportait pas de glycéride gélifié par le polymère cellulosique.

Le polymère cellulosique peut être choisi parmi les celluloses, le carboxyméthylcellulose, l'acétate de cellulose et l'hydroxyéthylcellulose. L'éthylcellulose est cependant préférée dans le cadre de la présente invention.

Le glycéride utilisé selon la présente invention, peut être choisi parmi les mono-, di- ou tri-glycérides synthétiques, semi-synthétiques ou d'origine naturelle, mais de préférence, on utilise le Labrafil ®. Le Labrafil ® est un glycéride que l'on peut gélifier par de l'éthylcellulose à haute température ; selon la présente invention, on choisit le Labrafil® M 1944CS, à savoir un mélange de glycérides oléiques polyoxyéthylénés obtenus par alcoolyse d'huile végétale naturelle (pharmacopée française 8ème édition).

Il s'agit d'un liquide huileux dont les caractéristiques sont représentées dans le tableau 1 suivant ; mais d'autres glycérides d'origine synthétique, semi-synthétique ou naturelle peuvent être utilisés.

2

TABLEAU I

Caractéristiques chimiques du Labrafil® M 1944CS
(Fabricant : Etablissements GATTEFOSSE)

| Nom chimique | Dénomination commerciale | Point de goutte °C | Indice de sapo. | Indice d'acide | Indice d'iode | Toxicite aigue orale/rat | ICP | HLB |
|---|---|---|---|---|---|---|---|---|
| Glycérides oléiques polyglycolisés (noyaux) | LABRAFIL M. 1944 CS | Liq. | 145/175 | < 2 | 60/90 | DL0 > 20ml/kg | 0 | 3/4 |

En variante, on peut aussi utiliser le Migliol ®, le Labrafac ®, le Transcutol ® et/ou des huiles naturelles.

L'éthylcellulose choisie de préférence selon l'invention est l'éthylcellulose Herculés®, à savoir un éther cellulosique préparé par réaction de chlorure d'éthyle avec de la cellulose alcaline ; il s'agit d'une poudre granulaire que l'on dépose en pluie dans la suspension huileuse de Labrafil® tout en agitant légèrement le mélange à une température pouvant atteindre 150°C sous atmosphère d'azote.

Aussitôt que l'éthylcellulose s'est totalement intégrée dans la suspension, on laisse refroidir le mélange par exemple jusqu'à 30°C, tout en maintenant une faible agitation. Le gel se forme ainsi progressivement.

Il suffit ensuite d'incorporer la doxycycline dans ce gel. Cette incorporation se fait grâce à une forte agitation. On utilise par exemple le mélangeur HOBART.

Enfin, il reste à conditionner la suspension. Le conditionnement est bien sûr adapté à la consistance et à la forme d'administration de la composition.

C'est ainsi que certaines compositions de la présente invention sont plus particulièrement appropriées à un conditionnement en seringues. Parmi celles-ci, on trouve notamment les gels comportant 2,5 % d'éthylcellulose Hercules ® additionné de 1 à 4 % de Migliol ® ou encore 2,5 % d'Ethocel Premium ® additionné de 1 à 4 % de préférence 2 % de Migliol ®.

Enfin, certains glycérides tels que les huiles de tournesol ou d'arachide peuvent être utilisées dans le cadre de la présente invention, mais nécessitent que les compositions correspondantes soient conditionnées à des températures comprises entre 60 et 150°C, de préférence de l'ordre de 130°C à cause d'une prise en masse rapide.

La composition pharmaceutique de la présente invention résoud tous les problèmes posés dans la technique antérieure.

En effet, une telle formulation utilisée en médecine humaine ou vétérinaire s'est révélée permettre une administration commode par voie orale, parentérale ou topique sans entraîner d'intolérance ou d'irritation ; en outre, une telle formulation peut être fortement dosée en principe actif.

La consistance de la composition, selon la présente invention, joue un rôle important pour l'obtention des avantages mentionnés ci-dessus.

La composition selon l'invention peut être plus ou moins pâteuse, voire solide. Elle est dans ce cas particulièrement bien adaptée pour être administrée par voie orale, topique, sous la forme d'un implant ou dans un système thérapeutique permettant la libération retardée du principe actif.

La composition de l'invention peut, au contraire, être plus ou moins fluide : il peut s'agir d'un gel, voire même d'un liquide, destinés plus particulièrement aux voies orales ou parentérales.

Le présent procédé de préparation peut être illustré par les exemples suivants :
- On incorpore 150 mg de doxycycline avec 1 ml d'huile de noyaux interestérifiée de type Labrafil ® préalablement gélifiée par 20 mg d'éthylcellulose. Le gel obtenu convient particulièrement à un usage parentéral.
- On incorpore 140 mg d'hyclate de doxycycline dans 1 ml d'huile de noyaux interestérifiée de type Labrafil ® préalablement gélifié par 40 mg d'éthylcellulose. La pâte obtenue peut être utilisée par voie orale.

On donne ci-après quelques exemples de formulations conformes à la présente invention (le Labrafil ® est, de préférence, du Labrafil ® M 1944CS) :

3

## 1 - PARACÉTAMOL

**Gel à 6 p.cent (P/P) de Paracétamol**

| Composition pour 1 kg : | |
|---|---|
| . Paracétamol | 60 g |
| . Ethylcellulose | 61 g |
| . Labrafil ® | 879 g |

Couleur     : blanc
Aspect      : homogène, consistance pâteuse
Densité     : environ 0,91

Remarque : Le Paracétamol n'est pas soluble à cette concentration dans l'eau et à fortiori n'est pas stable dans l'eau.

## 2 - PAMOATE DE PYRANTEL

**Gel à 4 p.cent (P/P) de pamoate de pyrantel**

| Composition pour 1 kg : | |
|---|---|
| . pamoate de pyrantel | 40 g |
| . Ethylcellulose | 63 g |
| . Labrafil ® | 897 g |

Couleur     : jaune clair
Aspect      : homogène, pâteux
Densité     : environ 0,97

**Gel à 12 p.cent (P/P) de pamoate de pyrantel**

| Composition pour 1 kg : | |
|---|---|
| . pamoate de pyrantel | 120 g |
| . Ethylcellulose | 58 g |
| . Labrafil ® | 822 g |

Couleur     : jaune clair
Aspect      : homogène, pâteux
Densité     : environ 0,95

3 - <u>AMOXYCILLINE</u>

**Gel à 4 p.cent (P/P) d'amoxycilline trihydratée**

| Composition pour 1 kg : | |
| --- | --- |
| . Amoxycilline trihydratée | 40 g |
| . Ethylcellulose | 63 g |
| . Labrafil ® | 897 g |

Couleur    : blanc
Aspect    : homogène, pâteux

4 - <u>DOXYCYCLINE BASE</u>

**Gel à 6,2 p.cent (P/P) de Doxycyline base**

| Composition pour 1 kg : | |
| --- | --- |
| . Doxycycline base | 62 g |
| . Ethylcellulose | 63 g |
| . Labrafil ® | 875 g |

Couleur    : brun
Aspect    : homogène, pâteux

**Suspension à 7 p.cent (P/P) de Doxycycline base**

| Composition pour 1 kg : | |
| --- | --- |
| . Doxycycline base | 70 g |
| . Ethylcellulose | 22 g |
| . Migliol ® | 30 g |
| . Labrafil ® | 878 g |

Couleur    : brun
Aspect    : homogène, fluide
Densité    : environ 0,92

5 - <u>HYCLATE DE DOXYCYCLINE</u>

**Gel à 6,5 p.cent (P/P) de Doxycycline**

| Composition pour 1 kg : | |
| --- | --- |
| . hyclate de doxycycline | 67 g |
| . Ethylcellulose | 47 g |
| . Labrafil ® | 886 g |

Couleur    : jaune clair
Aspect    : homogène, pâteux
Densité    : environ 0,92

EP 0 356 325 B1

**Gel à 17,5 p.cent (P/P) de Doxycycline**

| Composition pour 1 kg : | |
|---|---|
| . hyclate de doxycycline | 181 g |
| . Ethylcellulose | 85 g |
| . Labrafil ® | 733 g |

Couleur       : jaune clair
Aspect        : homogène, pâteux
Densité       : environ 0,95

Les différentes formulations ont bien sûr été testées en vue de connaître précisément deux de leurs caractéristiques essentielles dans le cadre de la présente invention, à savoir :
-   leur stabilité,
-   leur consistance adaptable à toutes les voies d'administration.

C'est ainsi que la stabilité d'un gel comportant en poids 7 % d'hyclate de doxycycline et 88 % de Labrafil ® M 1944 CS gélifié par 5 % d'éthylcellulose Hercules ® a été testée de la manière suivante (la concentration en hyclate de doxycycline est déterminée par chromatographie liquide haute performance et est exprimée en mg/g) :

| | Après 3 mois de conservation | Après 6 mois de conservation |
|---|---|---|
| **1. Etuve humide à 37°C** | | |
| Moyenne des 5 essais = | 68,2 | |
| % Variation = | 4,6 | |
| **2. Etuve sèche à 37°C** | | |
| Moyenne des 5 essais = | 73,4 | non déterminée |
| % Variation = | 2,8 | |
| **3. Température 45°C** | | |
| Moyenne des 5 essais = | 71,4 | |
| % Variation = | 2,0 | |
| (Très légère coloration brune) | | |
| **4. Température 4°C** | | |
| Moyenne des 3 essais = | 72,4 | 70,0 |
| % Variation = | 2,3 | 4,5 |
| **5. Température ambiante** | | |
| Moyenne des 5 essais = | 68,4 | 67,7 |
| % Variation = | 6,2 | 3,5 |

Il s'avère donc que, malgré les hautes températures et l'apparition d'une très légère coloration brune après stockage à une température supérieure à 37°C, il n'y a pas de diminution du titre en doxycycline.

En outre, le tableau 2 présente une comparaison des teneurs en épimères (en p. cent) de deux préparations contenant la doxycycline (sous forme hyclate), avec le principe actif ayant servi à leur

6

formulation. La formulation testée correspond à un produit stable.

EP 0 356 325 B1

TABLEAU 2

| 1°) Après 1 mois de conservation à 37°C (étuve sèche) | | |
|---|---|---|
| | Doxycycline hyclate | Gel à 6,5p.cent (P/P) |
| Doxycycline base (mg/g) | 858,2 | 63,4 |
| 6 Epidoxycycline** | 0,96 | 0,02 |
| Déméclocycline** | 1,99 | ND* |
| Oxytétracycline** | 0,65 | ND* |

| 2°) Après 3 mois de conservation à température ambiante | | | |
|---|---|---|---|
| | Doxycycline hyclate | Solution organique à 15p.cent | gel à 6,5p.cent |
| | | Lot 1     Lot 2 | (P/P) |
| Doxycycline base (mg/g) | 899,0 | 161,55     157,17 | 63,5 |
| 6 Epidoxycycline** | 0,30 | 0,98     1,02 | ND* |
| Déméclocycline** | 1,81 | ND*     ND* | ND* |
| Oxytétracycline base** | 0,43 | 0,12     0,20 | 0,02 |
| Méthacycline** | ND* | 1,34     1,57 | ND* |

*ND = Non Détectable

** = les teneurs en épimères sont exprimées en p.cent de la surface sous les pics chromatographiques. Elles représentent le rapport des aires sous les pics :

$$\frac{\text{épimère}}{\text{Doxycycline + somme des épimères}}$$

## TABLEAU 2 (suite)

| 3°) Après 6 mois de conservation à température ambiante | Gel à 6,5p.cent (P/P) |
|---|---|
| Doxycycline base (mg/g) | 66,1 |
| 6 Epidoxycycline** | 0,02 |
| Déméclocycline** | ND* |
| Oxytétracycline base** | 0,02 |
| Méthacycline** | 0,06 |

| 4°) Après 9 mois de conservation à température ambiante | Gel à 6,5p.cent (P/P) |
|---|---|
| Doxycycline base (mg/g) | 65,1 |
| 6 Epidoxycycline** | 0,31 |
| Déméclocycline** | ND* |
| Oxytétracycline base** | 0,06 |
| Méthacycline** | 1,41 |

La viscosité des compositions de la présente invention a aussi été déterminée et ce par l'étude de l'influence des diverses concentrations en dérivé cellulosique et en glycéride.

Les résultats obtenus sont reproduits dans le tableau 3 présenté ci-après.

Le tableau 3 représente la variation de la viscosité de l'huile gélifiée en fonction de la nature, de l'origine et de la teneur en dérivé cellulosique à température ambiante (le glycéride utilisé est le Labrafil ® M 1944CS).

TABLEAU 3

| Nature du dérivé cellulosique | Origine | % dans le gel (P/P) | C* | Consistance |
|---|---|---|---|---|
| éthylcellulose | Hercules® N-50 NF | 3,0 | 58,8 | fluide |
| " | " N-50 NF | 4,0 | 109,4 | pâteux |
| " | " | 5,0 | 182,2 | solide |
| " | " | 6,0 | 261,1 | solide |
| " | " | 7,0 | 261,1 | solide |
| " | " | 10,0 | ND | solide |
| éthylcellulose | Ethocel® 45 Premium | 3,0 | 64,6 | pâteux |
| " | " | 4,0 | 104,6 | pâteux |
| " | " | 5,0 | 182,2 | solide |
| " | " | 6,0 | 250,5 | solide |
| " | " | 7,0 | > 294,1 | solide |
| carboxyméthylcellulose | Déhydazol FK 62 | 3,0 | > 294,1 | gel non formé |
| méthylcellulose | Viscontran C3003 K300 | 3,0 | ND | sédimentation |

C* = viscosité en centipoises mesurée au viscomètre à cisaillement Brookfield avec un cône de diamètre D = 5 cm.

La gélification du Labrafac ® lipophile par 7 à 10 % d'éthylcellulose Hercules ® ne modifie pas la consistance des compositions reproduites dans le tableau 3.

En revanche, les suspensions comportant le Labrafac ® hydrophile gélifié par 7 ou 8 % d'éthylcellulose Herculès ® sont fluides. Si l'on augmente le pourcentage d'ethylcellulose, les suspensions comportant du Labrafac ® hydrophile deviennent pâteuses.

Enfin, le tableau 4 représente l'étude de la variation de la viscosité de la préparation contenant 6,5 p.cent de doxycycline en fonction de la température de stockage (mesurée à température ambiante).

**TABLEAU 4**

1 Mois après fabrication

| T° de stockage (°C) | Conditions de conservation | C | Consistance de la préparation |
|---|---|---|---|
| 37 | étuve sèche | 224 +/- 12 | solide |
| 37 | étuve humide | 229 +/- 23 | solide |
| 45 | étuve humide | 224 +/- 10 | solide |
| 20 | ambiante | 180 +/- 20 | solide |

Les compositions selon l'invention sont, en outre, compatibles avec une action prolongée du principe actif utilisé et notamment de la doxycycline.

C'est ainsi que des études entreprises chez l'animal, il ressort qu'une administration unique per os permet d'obtenir une concentration efficace en doxycycline pendant 40 à 84 heures.

Les résultats sont reproduits sur les tableaux 5 et 6 suivants.

Le tableau 5 représente les paramètres cinétiques chez l'animal du **gel à 6,5 p.cent (P/P) de Doxycycline** (sous forme hyclate), après administration unique par voie orale :

Tableau 5

| Espèce | Dose mg/kg | Cmax | Tmax | T 1/2 B | F |
|--------|-----------|------|------|---------|---|
| Bovine | 20 | 6,2 | 4-10 | 22 | 77,4 + 9,3 |
| Canine | 10 | 2,9 | 4-8 | 19 | Non déterminé |
| Cmax : concentration au pic en ug/ml de plasma, déterminée par HPLC par la méthode de RIOND et al. | | | | | |
| Tmax : temps au pic mini-maxi en heures. | | | | | |
| T 1/2 B : temps de demi-élimination en heures (modèle à 2 compartiments). | | | | | |
| F : biodisponibilité absolue mesurée sur 4 sujets en p.cent. | | | | | |

Le tableau 6 représente les paramètres cinétiques chez les bovins du **gel à 6,5 p.cent (P/P) de Doxycycline** (sous forme hyclate, lot d'essai) après administration unique par voie sous-cutanée :

Tableau 6

| Dose mg/kg | Cmax | Tmax | T 1/2 B |
|-----------|------|------|---------|
| 20 | 1 | 2 | 40 |
| Cmax : concentration au pic en g/ml de plasma, déterminée par HPLC par la méthode de FOURTILLAN et al. Now. Presse Med 1980 ; 2 : 77-81. | | | |
| Tmax, T 1/2 : idem tableau précédent. | | | |

De même, les études pharmacologiques et toxicologiques d'un gel de doxycycline conforme à la présente invention, ont été entreprises chez le chien, le veau et le porc. Les résultats ne sont pas reproduits intégralement ici, afin d'alléger la présente description.

De ces études, il ressort que la cinétique d'un tel gel est très favorable à une administration thérapeutique efficace chez l'homme ou l'animal : inertie métabolique et absence de modification de la biodisponibilité.

EXEMPLE 1 **Densité des compositions de la présente invention**

I - Hyclate de Doxycline + Labrafil ® + éthylcellulose

A. Formule de fabrication

Préparation pour 4 kg de suspension à 700 mg de principe actif pour 10 ml de suspension et à 5 % d'Ethylcellulose Hercules ® :

| | |
|---|---|
| - Labrafil ® | 3 800 g |
| - Ethylcellulose | 200 g |
| - Doxycycline hyclate | 280 g |

B. Mode opératoire

- Mélanger le Labrafil ® et l'Ethylcellulose Hercules ® sous azote, jusqu'à une température de 150°C, afin d'obtenir un liquide visqueux et limpide,

11

- Laisser refroidir jusqu'à 30°C et incorporer l'hyclate de doxycycline sous agitation,
- Effectuer le conditionnement en seringues dès la fin de l'incorporation du principe actif.

C. Calcul de densité

Poids P1 :    peser une fiole jaugée de 50 ml + un agitateur + eau distillée.
q.s.p. 50 ml.
P1 = 84,521

Poids P2 :    peser 10 g de gel dans la même fiole. P2 = 10,1062 g

Poids P3 :    ajouter le même agitateur et compléter à 50 ml avec de l'eau distillée. P3 = 83,750 g

$$\text{Densité} = D = \frac{P2}{P1 - (P3 - P2)}$$

d'où

$$D = \frac{10,1062}{84,521 - (83,750 - 10,1062)} = 0,929$$

II - Doxycycline base + Ethylcellulose Hercules ® 5 % + Aérosil ® 3 %

Poids P1 :    peser une fiole jaugée de 50 ml + un agitateur + eau distillée
q.s.p. 50 ml.
P1 = 81,593 g.

Poids P2 :    peser 10 g de gel dans la même fiole.
P2 = 10,0894 g.

Poids P3 :    ajouter le même agitateur et compléter à 50 ml avec de l'eau distillée.
P3 = 80,772 g.

d'où

$$\text{densité} = D = \frac{P2}{P1 - (P3 - P2)} = 0,9247$$

III - Doxycycline base + Ethylcellulose Hercules 2,5 % + Aérosil 3 %

Poids P1 :    peser une fiole jaugée de 50 ml + un agitateur + eau distillée.
q.s.p. 50 ml.
P1 = 96,424 g.

Poids P2 :    peser 10 g de gel dans la même fiole.
P2 = 10,349 g.

Poids P3 :    ajouter le même agitateur et compléter à 50 ml avec de l'eau distillée.
P3 = 95,488 g.

d'où

$$\text{densité} = D = \frac{P2}{P1 - (P3 - P2)} = 0,917$$

IV - <u>Suspension à 7 % d'Ethylcellulose Hercules ® + 40 g de pamoate de pyrantel par kg de gel</u>

De la même façon :
P1 = 95,284 g,
P2 = 9,9964 g,
P3 = 94,965 g,
d'où D = 0,969.

V - <u>Suspension à 7 % d'Ethylcellulose Hercules ® et à 60 g de paracétamol par kg de gel</u>

De la même façon :
P1 = 96,520 g,
P2 = 9,9903 g,
P3 = 95,556 g,
d'où D = 0,9119.

VI - <u>Suspension à 7 % d'Ethylcellulose Hercules ® et à 120 g de pamoate de pyrantel par kg de gel</u>

De la même façon : P1 = 79,739 g,
P2 = 10,2935 g,
P3 = 79,219 g,
d'où D = 0,952.

<u>EXEMPLE 2</u> **Détermination d'un taux optimum en éthylcellulose pour la réalisation d'une suspension injectable de doxycycline**

Le but de l'étude est la réalisation d'une suspension injectable contenant de la doxycycline, celle-ci ayant un temps de libération pouvant aller de 7 à 14 jours avec un minimum de risques pour les animaux receveurs. On choisit, à titre de véhicule pour cette suspension, un glycéride semi-synthétique, gélifiable par de l'éthylcellulose à haute température.

Parmi tous les glycérides semi-synthétiques présentant une tolérance et une qualité satisfaisantes, on choisit le Labrafil ® M 1944 CS des Ets Gattefossé.

Quant à l'éthylcellulose, on choisit d'utiliser une éthylcellulose Hercules ® de qualité N50NF.

Au cours de la première étape de préparation de la suspension, la formation d'un gel à 2,5 %, 4 % et 5 %, a été réalisée dans les conditions de préparation ci-après : après pesée des composants, le Labrafil ® est placé dans un mélangeur pouvant atteindre la température de 150°. On dépose l'éthylcellulose en pluie et, on agite lentement tout en élevant la température à 150° sous azote. Une fois que l'ethylcellulose s'est totalement intégrée dans la suspension, on laisse refroidir à 30°C. A cette température, le gel est formé.

Dans une deuxième étape, la doxycycline est agitée dans le même mélangeur en fonction de la quantité de gel préparée et, par agitation régulière non destructrice, la mise en suspension de la doxycycline s'effectue. Aucune autre opération n'est nécessaire sauf éventuellement, un broyage ou une homogénéisation sur le plan industriel.

Le gel ainsi obtenu est ensuite conditionné dans des seringues en matière plastique, munies, pour la circonstance, de petites aiguilles à injection servant à boucher l'extrêmité de la seringue. Le remplissage industriel s'effectuera pratiquement dans les mêmes conditions avec une pompe doseuse.

Plusieurs formules ont été réalisées avec 7 % et 14 % en poids de principe actif.

Les études de l'aptitude au conditionnement en seringue ont montré que les gels à 2,5 %, titrant 7 % et 14 % en poids de principe actif passaient bien à travers l'aiguille, mais, que les gels à 5 % dosés à 7 % en principe actif, passaient plus difficilement.

La préparation d'un gel à 4 % n'améliore pas les qualités d'aptitude au conditionnement en seringue. Le taux de 2,5 % en éthylcellulose paraît donc être le plus intéressant.

**Revendications**

1. Composition pharmaceutique stable administrable par voie orale, topique ou parentérale chez l'homme ou l'animal, caractérisée en ce qu'elle comporte un principe actif peu soluble dans l'eau, à raison d'une quantité pouvant atteindre 25 % en poids, et au moins un glycéride gélifié par au moins un polymère cellulosique.

**2.** Composition selon la revendication 1, caractérisée en ce que la quantité en polymère cellulosique est comprise entre 1 et 10 % en poids.

**3.** Composition selon la revendication 1 ou 2, caractérisée en ce qu'elle comporte à titre de principe actif la doxycycline ou l'un de ses sels.

**4.** Composition selon la revendication 3, caractérisée en ce que le sel de doxycycline est l'hyclate.

**5.** Composition selon la revendication 1 ou 2, caractérisée en ce qu'elle comporte, à titre de principe actif, un composé choisi parmi le paracétamol, le pamoate de pyrantel, l'amoxycilline et les mélanges vitaminiques.

**6.** Composition selon l'une quelconque des revendications 1 à 5, caractérisée en ce que le polymère cellulosique est l'éthylcellulose ou la carboxyméthylcellulose.

**7.** Composition selon l'une quelconque des revendications 1 à 6, caractérisée en ce que le glycéride est choisi parmi le Labrafil®, le Migliol ® , le Labrafac ®, le Transcutol ®, et/ou des huiles naturelles.

**8.** Composition selon l'une quelconque des revendications 1 à 7, caractérisée en ce qu'elle présente une consistance pâteuse ou solide.

**9.** Composition selon l'une quelconque des revendications 1 à 7, caractérisée en ce qu'elle présente une consistance liquide ou de gel.

**10.** Composition selon l'une quelconque des revendications 8 ou 9, caractérisée en ce qu'elle comporte en poids 3 à 20 % d'hyclate de doxycycline, 0,5 à 10 % d'éthylcellulose et 25 à 95 % de Labrafil ®.

**11.** Composition injectable selon la revendication 9, caractérisée en ce qu'elle comporte en poids 1 à 40 % de Migliol ® gélifié par 2,5 % d'éthylcellulose Hercules ® ou par 2,5 % d'Ethocel Premium ®.

**Claims**

**1.** Stable pharmaceutical composition which can be administered orally, topically or parenterally to humans or animals, characterized in that it contains a sparingly water-soluble active principle in the proportion of an amount which can be as high as 25 % by weight, and at least one glyceride gelled with at least one cellulose polymer.

**2.** Composition according to Claim 1, characterized in that the amount of cellulose polymer is between 1 and 10 % by weight.

**3.** Composition according to Claim 1 or 2, characterized in that it contains doxycycline or one of its salts as active principle.

**4.** Composition according to Claim 3, characterized in that the doxycycline salt is the hyclate salt.

**5.** Composition according to Claim 1 or 2, characterized in that it contains, as active principle, a compound chosen from paracetamol, pyrantel pamoate, amorycillin and vitamin mixtures.

**6.** Composition according to any one of Claims 1 to 5, characterized in that the cellulose polymer is ethyl cellulose or carboxymethyl cellulose.

**7.** Composition according to any one of Claims 1 to 6, characterized in that the glyceride is chosen from Labrafil®, Miglyol®, Labrafac®, Transcutol® and/or natural oils.

**8.** Composition according to any one of Claims 1 to 7, characterized in that it possesses a pasty or solid consistency.

14

**9.** Composition according to any one of Claims 1 to 7, characterized in that it possesses a liquid or gel consistency.

**10.** Composition according to either of Claims 8 and 9, characterized in that it contains, by weight, 3 to 20 % of doxycycline hyclate, 0.5 to 10 % of ethyl cellulose and 25 to 95 % of Labrafil®.

**11.** Injectable composition according to Claim 9, characterized in that it contains, by weight, 1 to 40 % of Miglyol® gelled with 2.5 % of Hercules® ethyl cellulose or with 2.5 % of Ethocel Premium®.

**Patentansprüche**

**1.** Stabile pharmazeutische Zusammensetzung, die auf oralem, topischem oder parenteralem Wege an Mensch oder Tier verabreichbar ist, dadurch gekennzeichnet, daß sie enthält einen in Wasser wenig löslichen Wirkstoff (aktives Prinzip) in einer Menge, die 25 Gew.-% erreichen kann, und mindestens ein Glycerid, das durch mindestens ein Cellulose-Polymer geliert worden ist.

**2.** Zusammensetzung nach Anspruch 1, dadurch gekennzeichnet, daß die Menge an Cellulose-Polymer zwischen 1 und 10 Gew.-% liegt.

**3.** Zusammensetzung nach Anspruch 1 oder 2, dadurch gekennzeichnet, daß sie als Wirkstoff (aktives Prinzip) Doxycyclin oder eines seiner Salze enthält.

**4.** Zusammensetzung nach Anspruch 3, dadurch gekennzeichnet, daß es sich bei dem Salz von Doxycyclin um das Hyclat handelt.

**5.** Zusammensetzung nach Anspruch 1 oder 2, dadurch gekennzeichnet, daß sie als Wirkstoff (aktives Prinzip) eine Verbindung, ausgewählt aus Paracetamol, Pyrantelpamoat, Amoxycillin und Vitaminmischungen, enthält.

**6.** Zusammensetzung nach einem der Ansprüche 1 bis 5, dadurch gekennzeichnet, daß es sich bei dem Cellulose-Polymer um Ethylcellulose oder Carboxymethylcellulose handelt.

**7.** Zusammensetzung nach einem der Ansprüche 1 bis 6, dadurch gekennzeichnet, daß das Glycerid ausgewählt wird aus Labrafil ®, Migliol ®, Labrafac ®, Transcutol ® und/oder natürlichen Ölen.

**8.** Zusammensetzung nach einem der Ansprüche 1 bis 7, dadurch gekennzeichnet, daß sie eine pastöse oder feste Konsistenz aufweist.

**9.** Zusammensetzung nach einem der Ansprüche 1 bis 7, dadurch gekennzeichnet, daß sie eine flüssige oder Gel-Konsistenz aufweist.

**10.** Zusammensetzung nach einem der Ansprüche 8 oder 9, dadurch gekennzeichnet, daß sie 3 bis 20 Gew.-% Doxycyclinhyclat, 0,5 bis 10 Gew.-% Ethylcellulose und 25 bis 95 Gew.-% Labrafil ® enthält.

**11.** Injizierbare Zusammensetzung nach Anspruch 9, dadurch gekennzeichnet, daß sie 1 bis 40 Gew.-% Migliol ®, geliert mit 2,5 % Ethylcellulose Hercules ® oder mit 2,5 % Ethocel Premium ®, enthält.